# FASCICULE DE BREVET EUROPEEN

(11) **EP 3 119 889 B1**
(45) Date de publication et mention de la délivrance du brevet: **24.02.2021**
(21) Numéro de dépôt: 15717557.1
(22) Date de dépôt: 20.03.2015
(51) Int. Cl.: C12N 15/115, A61K 31/7088, C12Q 1/68, A61P 35/02

(54) **INHIBITEURS DE STAT5 ET UTILISATION DE CEUX-CI**
STAT5-HEMMER UND VERWENDUNG DAVON
STAT5 INHIBITORS AND USE OF SAME

(30) Priorité: 20.03.2014 FR 1452345
(43) Date de publication de la demande: 25.01.2017
(73) Titulaire: Centre National de la Recherche Scientifique (CNRS), 75016 Paris (FR); UTC (Université de Technologie de Compiègne), 60203 Compiègne cedex (FR)
(72) Inventeur: AVALLE-BIHAN, Bérangère, 60200 Compiègne (FR); LOUSSOUARN, Claire, 60880 Le Meux (FR); ISBER, Hassan, 60200 Compiègne (FR); FRIBOULET, Alain, 60680 Jonquières (FR); PADIOLLEAU, Séverine, 60127 Morienval (FR)
(74) Mandataire: Icosa
(86) Numéro de dépôt international: PCT/FR2015/050711
(87) Numéro de publication internationale: WO 2015/140479

(56) Documents cités:
- LOUSSOUARN C ET AL: "Selection of DNA aptamers regulating STAT5B, a protein involved in leukemias", FEBS JOURNAL, vol. 280, no. Suppl. 1, Sp. Iss. SI, juillet 2013 (2013-07), page 320, XP002729464, & 38TH CONGRESS OF THE FEDERATION-OF-EUROPEAN-BIOCHEMICAL-SOCIETI ES (FEBS); SAINT PETERSBURG, RUSSIA; JULY 06 -11, 2013
- E. A. NELSON ET AL: "The STAT5 Inhibitor Pimozide Displays Efficacy in Models of Acute Myelogenous Leukemia Driven by FLT3 Mutations", GENES & CANCER, vol. 3, no. 7-8, 1 juillet 2012 (2012-07-01), pages 503-511, XP055137993, ISSN: 1947-6019, DOI: 10.1177/1947601912466555 cité dans la demande
- BRENT D. G. PAGE ET AL: "Small Molecule STAT5-SH2 Domain Inhibitors Exhibit Potent Antileukemia Activity", JOURNAL OF MEDICINAL CHEMISTRY, vol. 55, no. 3, 9 février 2012 (2012-02-09), pages 1047-1055, XP055137989, ISSN: 0022-2623, DOI: 10.1021/jm200720n cité dans la demande
- F. BEHBOD ET AL: "Specific Inhibition of Stat5a/b Promotes Apoptosis of IL-2-Responsive Primary and Tumor-Derived Lymphoid Cells", THE JOURNAL OF IMMUNOLOGY, vol. 171, no. 8, 6 octobre 2003 (2003-10-06), pages 3919-3927, XP055137991, ISSN: 0022-1767, DOI: 10.4049/jimmunol.171.8.3919
- BENEKLI MUSTAFA ET AL: "Signal transducer and activator of transcription proteins in leukemias.", BLOOD, vol. 101, no. 8, 15 avril 2003 (2003-04-15), pages 2940-2954, XP055137992, ISSN: 0006-4971 cité dans la demande
- STOLTENBURG R ET AL: "FluMag-SELEX as an advantageous method for DNA aptamer selection", ANALYTICAL AND BIOANALYTICAL CHEMISTRY, vol. 383, 29 juillet 2005 (2005-07-29), pages 83-91, XP002408446, ISSN: 1618-2642, DOI: 10.1007/S00216-005-3388-9 cité dans la demande
- AXEL WEBER ET AL: "The Inhibition of Stat5 by a Peptide Aptamer Ligand Specific for the DNA Binding Domain Prevents Target Gene Transactivation and the Growth of Breast and Prostate Tumor Cells", PHARMACEUTICALS, vol. 6, no. 8, 20 août 2013 (2013-08-20), pages 960-987, XP055138067, DOI: 10.3390/ph6080960

## Description

### DOMAINE DE L'INVENTION

La présente invention concerne le traitement du cancer, en particulier des leucémies via l'inhibition de la protéine STAT5. La présente invention concerne plus particulièrement des aptamères spécifiques de la protéine STAT5, et leur utilisation thérapeutique ou diagnostique.

### ÉTAT DE LA TECHNIQUE

L'objectif de cette invention est de proposer une nouvelle molécule qui pourrait entrer dans un processus thérapeutique des cancers, en particulier des leucémies et les néoplasmes myéloprolifératifs. Les leucémies et les néoplasmes myéloprolifératifs sont actuellement les cas de cancer à forte mortalité les plus fréquents chez les hommes de moins de 40 ans et les femmes de moins de 20 ans. Un des marqueurs reconnus de ces maladies est le dysfonctionnement du facteur de transcription (FT) Signal Transducer and Activator of Transcription 5 (STAT5) qui se réfère à deux protéines, STAT5A et STAT5B qui appartiennent à la famille des protéines STAT composée de 7 membres. Les protéines STAT5A et STAT5B sont codées par 2 gènes distincts mais leur séquence d'acides aminés est identique à plus de 90%. Les protéines STAT5 sont impliquées dans des voies de signalisation cytosoliques. Des études ont démontré qu'un dysfonctionnement de l'activité de STAT5A et STAT5B pourrait contribuer à l'induction de cancers humains.

L'activation des protéines STAT5B est fréquemment retrouvée dans des cas d'hémopathies malignes. Dans ce contexte, la protéine STAT5B a été mise en évidence comme étant responsable de la leucémogénèse (Benekli et al., 2003. Blood. 101(8):2940-54). Des études ont également montré que les facteurs de transcription STAT5 étaient activés par un large spectre de ligands leur permettant d'intervenir dans la régulation physiologique et physiopathologique de grandes fonctions biologiques telles que la prolifération cellulaire, la différenciation cellulaire ou encore l'apoptose. La protéine STAT5B constitue donc une cible prometteuse pour de nouveaux traitements anti-cancéreux. D'un point de vue applicatif, l'obtention de molécules inhibant l'activité de la protéine STAT5B est d'autant plus importante que l'on sait que les traitements actuels sont peu spécifiques et entraînent des effets secondaires indésirables.

En effet, les traitements tels que la radiothérapie ou la chimiothérapie sont des traitements lourds qui nécessitent de longues hospitalisations dans certains cas. Certains patients développent une résistance à l'inhibiteur pharmacologique Imatinib mesylate (ou Glivec® - Novartis) utilisé actuellement en première intention en clinique dans certains cas de leucémies. Il est donc indispensable de développer de nouveaux composés permettant de traiter ces maladies.

Des inhibiteurs de STAT5 sont déjà développés tels que AZD1480 mais il est également un inhibiteur de STAT3 et présente des effets secondaires. Le pimozide inhibe STAT5 mais est également un antagoniste aux récepteurs de la dopamine et par conséquent un antipsychotique (Nelson et al., 2012. Genes Cancer. 3(7-8):503-11). Des antisens pour STAT5 A/B ont été testés *in vitro* dans des travaux fondamentaux mais n'ont pas encore été développés ni testés dans des essais cliniques (Behbod et al., 2003. J Immunol. 171(8):3919-27 ; Futami et al., 2008. Leukemia. 22(6):1131-8 ; Page et al., 2011. JMed Chem. 55(3):1047-55).

Dans la présente invention, de nouveaux aptamères spécifiques de STAT5 sont décrits. Ces aptamères présentent l'avantage d'inhiber la protéine STAT5 le plus en aval possible de la voie de signalisation afin d'éviter des effets secondaires indésirables qui pourraient être liés à l'inhibition de l'expression de la protéine STAT5. En effet, la protéine STAT5 est impliquée dans des processus divers et à différents niveaux dans les voies de signalisation et son inhibition globalisée pourrait être délétère pour la cellule. Ces molécules présentent également l'avantage d'être hautement spécifiques de la protéine ciblée, et faiblement immunogènes, et démontrent des propriétés anti-prolifératives et pro-apoptotiques efficaces (voir les exemples).

### RÉSUMÉ

Un objet de l'invention est un aptamère d'acides nucléiques se liant spécifiquement à STAT5, de préférence à STAT5B, ledit aptamère étant caractérisé en ce qu'il comprend la séquence SEQ ID NO : 1 ou SEQ ID NO : 2 ou SEQ ID NO : 57, ou un variant ayant au moins 90% d'identité de séquence avec SEQ ID NO : 1 ou SEQ ID NO : 2 ou SEQ ID NO : 57.

Dans un mode de réalisation, ledit aptamère comprend en outre un groupement additionnel de stabilisation et/ou un groupement additionnel pour vectorisation.

Un autre objet de l'invention est une composition pharmaceutique comprenant au moins un aptamère tel que décrit ci-dessus et un excipient pharmaceutiquement acceptable.

Un autre objet de l'invention est un médicament comprenant au moins un aptamère tel que décrit ci-dessus.

L'invention a également pour objet un aptamère, une composition pharmaceutique ou un médicament tels que décrits ci-dessus pour leur utilisation dans le traitement de cancers, de préférence de leucémies.

Dans un mode de réalisation, l'aptamère ou la composition pharmaceutique ou le médicament de l'invention est utilisé en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, des inhibiteurs les tyrosines kinases, les agents différenciants, ou un mélange de ceux-ci.

Dans une autre mode de réalisation, l'aptamère de l'invention est en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, les inhibiteurs des tyrosines kinases, les agents différenciants, ou un mélange de ceux-ci, pour son utilisation dans le traitement de cancers, de préférence de leucémies.

Dans une autre mode de réalisation, la composition pharmaceutique ou le médicament de l'invention est en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, les inhibiteurs des tyrosines kinases, les agents différenciants, ou un mélange de ceux-ci, pour son utilisation dans le traitement de cancers, de préférence de leucémies.

L'invention a également pour objet un procédé de détection de STAT5 dans un échantillon biologique comprenant :
a. la mise en contact de l'aptamère tel que décrit ci-dessus avec ledit échantillon préalablement prélevé sur un sujet,
b. la détermination de la quantité dudit aptamère lié audit échantillon.

### DÉFINITIONS

Dans la présente invention, les termes ci-dessous sont définis de la manière suivante :
- **"Aptamère"** concerne un oligonucléotide isolé et peut aussi indifféremment être désigné dans la présente invention « aptamère d'acide nucléique ». L'aptamère ou l'aptamère d'acide nucléique est une séquence oligonucléotidique simple-brin ou double-brin qui est capable de se lier à une protéine ou autre molécule, et ainsi perturbant la fonction de ladite protéine ou autre molécule. Dans un mode de réalisation, l'acide nucléique comprend des unités ribonucléosides.
- Au sens de la présente invention, les termes **"traitement", "traiter"** ou **"soulager"** concernent à la fois le traitement thérapeutique et les mesures prophylactiques ou préventives, dont l'objet est de prévenir ou de retarder l'apparition ou l'installation d'un cancer. Les sujets à traiter incluent donc à la fois les sujets déjà atteints d'un cancer, et les sujets prédisposés à un cancer ou les sujets chez lesquels une telle maladie doit être prévenue. Un sujet est efficacement « traité » d'un cancer si, après avoir reçu une quantité thérapeutiquement efficace d'un aptamère selon l'invention, ledit sujet montre une amélioration observable et/ou mesurable dans le nombre de cellules cancéreuses, et/ou une amélioration notable de sa qualité de vie. Ces paramètres d'évaluation d'un traitement efficace sont facilement mesurables par des procédures de routine familières à l'homme du métier.

- Au sens de la présente invention, l'expression **"quantité efficace"** (ou **"quantité thérapeutiquement efficace**") se réfère à une quantité de l'aptamère selon l'invention nécessaire ou suffisante pour, sans causer d'effets secondaires significatifs et défavorables pour le sujet, (1) retarder ou stopper l'apparition d'un cancer, (2) apporter des améliorations, (3) réduire la sévérité ou l'incidence d'un cancer, ou (4) stopper ou soigner un cancer. Une quantité efficace peut être administrée avant l'apparition d'un cancer, pour une action prophylactique ou préventive. De façon alternative ou additionnelle, une quantité efficace peut être administrée après l'apparition d'un cancer, pour une action thérapeutique.
- Un **"excipient"** désigne, dans la présente invention, toute substance autre que le principe actif présente dans une composition lui conférant des propriétés de stabilité, de forme (liquide, solide, gélule, etc... suivant le mode d'administration), de goût, de dissolution (par exemple dissolution ciblée dans l'estomac ou le tube digestif), de couleur, etc... Un **"excipient pharmaceutiquement acceptable"** désigne plus spécifiquement un excipient qui n'induit pas de réaction allergique ou non-souhaitée lors de son administration à un sujet, de préférence à un humain. Cette définition inclut tous les solvants, milieux de dispersion, enrobages, agents antibactériens ou antifongiques, agents isotoniques et agents permettant de retarder l'absorption du principe actif, etc. Pour une administration à l'homme, les préparations doivent remplir les conditions de stérilité, de pyrogénicité, de sécurité générale et les standards de pureté définis par le bureau des normes biologiques de la FDA.
- **"Environ",** placé devant un nombre, signifie plus ou moins 10% de la valeur nominale de ce nombre.
- **"Spécifique":** qui se lie spécifiquement à la protéine ciblée et qui déclenche un effet biologique ou au contraire bloque l'effet biologique de la protéine ciblée. Cette liaison est saturable alors qu'une liaison non-spécifique ne déclenche aucun effet biologique mesurable et non saturable. Un aptamère est dit se lier de manière spécifique à une cible lorsqu'il ne présente essentiellement pas d'affinité pour un composé sans rapport structural avec la cible. De préférence, dans le cas d'une cible protéique, un composé protéique est dit sans rapport structural avec la cible selon l'invention, lorsque l'identité de séquence entre la cible et le composé est inférieure à 60%, de préférence inférieure à 70%, de préférence encore inférieure à 80%.

### DESCRIPTION DÉTAILLÉE

La présente invention concerne des aptamères d'acides nucléiques se liant spécifiquement à STAT5.

L'interaction spécifique entre un aptamère et la protéine cible peut être déterminée par le Test A tel que décrit dans l'exemple 2 de la présente invention.

200 pmol d'aptamères biotinylés sont incubés avec 200 µg de protéines des extraits cytoplasmiques et nucléaires pendant 2h sur glace dans le tampon de liaison/lavage (5X : Tris pH 7,5 50 mM ; NaCl 50 mM ; EDTA 5 mM ; PMSF 2,5 mM ; Glycérol 25% ; NP40-Tergitol® 0,5%). Le mélange est ensuite mis en contact avec 100 µL de billes streptavidine (Dynal) pendant 30 minutes sous agitation à 4°C. Le tout est lavé 3 fois avec le tampon de lavage. Les protéines sont ensuite éluées en ajoutant 40 µL de tampon d'élution (Tris 0,5 M 250 mM pH 6,8 ; glycérol 25% ; SDS 8% ; β-mercaptoéthanol 20% ; H₂O qsp 10 mL) et en chauffant 5 minutes à 90°C.

Les protéines sont analysées par Western Blot après migration électrophorétique sur gel SDS-PAGE.

Les protéines STAT5 activées sont détectées en exploitant deux anticorps (aux concentrations indiquées par le fournisseur) : anticorps anti-STAT5 (C-17 Santa Cruz) produit chez le lapin ; anticorps anti-PhosphoSTAT5 (Y694) produit chez le lapin (Cell Signaling).

La reconnaissance spécifique de ces anticorps est ensuite révélée par un anticorps secondaire (anti IgG de lapin) marqué à la peroxydase (Sigma-Aldrich) dilué au 5000^{ème}. Dans un mode de réalisation, un aptamère est dit ne présenter essentiellement pas d'affinité pour un composé selon l'invention, notamment lorsque la constante de dissociation (K_{D}) de l'aptamère vis-à-vis d'une protéine est supérieure à 10⁻⁶ mol/L, de préférence supérieure à 10⁻⁷ mol/L. La constante de dissociation peut notamment être déterminée, dans des conditions standards, à l'aide des représentations de Scatchard et de Lineweaver Burk bien connues de l'homme du métier. De préférence, l'affinité de l'aptamère de l'invention pour une protéine est d'un K_{D} d'environ 100 pM à environ 10 nM.

Dans un mode de réalisation, l'aptamère de l'invention module la voie de signalisation STAT/Jak (Janus Kinase). La dérégulation de la voie de signalisation STAT/Jak est fortement impliquée dans le développement de cancers. La dérégulation de la voie de signalisation STAT/Jak est bien connue de l'homme du métier et mesurable en Western-Blot ou tests ELISA permettant de déterminer le niveau de phosphorylation des kinases impliquées dans cette voie.

Selon un mode de réalisation, les aptamères de l'invention se lient spécifiquement à la protéine STAT5A humaine (SEQ ID NO : 5) et la protéine STAT5B humaine (SEQ ID NO : 6), de préférence STAT5B. Selon un autre mode de réalisation, les aptamères de l'invention inhibent, de préférence inhibent spécifiquement, STAT5A (SEQ ID NO : 5) et STAT5B (SEQ ID NO : 6), de préférence STAT5B.

Comme la protéine STAT5 intervient à différents niveaux de la voie de transduction du signal, plusieurs possibilités d'inhibition sont possibles. Dans un mode de réalisation, STAT5 est inhibée au niveau du cytoplasme lorsque la protéine est sous forme monomérique. Dans un autre mode de réalisation, l'inhibition intervient au cours du processus de dimérisation et/ou lors de la translocation de celle-ci vers le noyau empêchant ainsi sa fixation au niveau de ses séquences cible. Dans un autre mode de réalisation, l'inhibition de STAT5 intervient pour empêcher sa fixation sur le promoteur d'un gène au niveau de l'ADN. Dans un autre mode de réalisation, l'inhibition de STAT5 correspond à l'inhibition de sa phosphorylation, et peut être mesurée par des techniques bien connues de l'homme du métier, telle que, par exemple, un Western-Blot avec par exemple, les Anticorps anti-STAT5 (C-17 Santa Cruz) produit chez le lapin ; Anticorps anti-PhosphoSTAT5 (Y694) produit chez le lapin (Cell Signaling).

L'aptamère de la présente invention comprend ou consiste en une séquence sélectionnée parmi la séquence Apta 1 (SEQ ID NO : 1), la séquence Apta 2 (SEQ ID NO : 2), la séquence Apta 3 (SEQ ID NO : 57), un variant de Apta 1, un variant de Apta 2 ou un variant de Apta 3.

SEQ ID NO 1 : 5’-
TATCCGCAACCCACCTAGCGCCCTACCTCGTGGGAATCCAAACCCAACCAG
TCCACCCAC -3’

SEQ ID NO : 2 : 5’-
GTGTCTGTTCACTCGTCGATACACAGCATACTCAACCCAGGCCCCTGACTGC
TAATCCCC -3’

SEQ ID NO : 57 : 5'-
GTGTCTGTTCACTCGTCGATACACAACATACTCAACCCAGGCCCCTGACTGC
TAATCCCC-3'.

Dans un mode de réalisation, l'aptamère de la présente invention comprend ou consiste en une séquence sélectionnée parmi les séquences Apta 1, Apta 2 et Apta 3, ou un variant de celles-ci encadrée en 5' et 3' par une séquence flanquante.

Dans un mode de réalisation, l'aptamère de la présente invention comprenant SEQ ID NO : 1 ou SEQ ID NO : 2 ou SEQ ID NO : 57 selon l'invention peut notamment comprendre des séquences du côté 5' et/ou 3' visant à structurer l'acide nucléique telles des séquences flanquantes. Préférentiellement, l'aptamère selon l'invention comprend, ou est constitué de SEQ ID NO : 3 ou 4 ou 58, qui comprennent respectivement SEQ ID NO : 1 et 2 et 57. Dans ce mode de réalisation, l'invention concerne alors également, en particulier, un aptamère comprenant, ou constitué d'au moins 15 nucléotides consécutifs d'une séquence ayant au moins 60% d'identité avec SEQ ID NO : 3 ou SEQ ID NO : 4 ou SEQ ID NO : 58, sous réserve qu'un aptamère constitué de cette séquence présente se lie spécifiquement à STAT5.

SEQ ID NO : 3 : 5’- ATACCAGCTTATTCAATT
TATCCGCAACCCACCTAGCGCCCTACCTCGTGGGAATCCAAACCCAACC
AGTCCACCCACAGATAGTAAGTGCAATCT -3’

SEQ ID NO : 4 : 5'- ATACCAGCTTATTCAATT
GTGTCTGTTCACTCGTCGATACACAGCATACTCAACCCAGGCCCCTGAC
TGCTAATCCCCAGATAGTAAGTGCAATCT -3'

SEQ ID NO : 58 : 5'- ATACCAGCTTATTCAATT
GTGTCTGTTCACTCGTCGATACACAACATACTCAACCCAGGCCCCTGAC
TGCTAATCCCCAGATAGTAAGTGCAATCT-3'

Dans un autre mode de réalisation, le variant de l'aptamère de l'invention comprend ou consiste en une séquence ayant au moins 90%, 95%, 96%, 97%, 98%, ou 99% d'identité avec SEQ ID NO : 1 ou 2 ou 57.

Dans un autre mode de réalisation, le variant de l'aptamère de l'invention comprend ou consiste en une séquence d'au moins 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 ou 50 nucléotides ayant au moins 90%, 95%, 96%, 97%, 98%, ou 99% d'identité avec SEQ ID NO : 1 ou 2 ou 57.

Selon un autre mode de réalisation, une séquence ayant au moins 60% d'identité en nucléotides avec SEQ ID NO : 1 ou SEQ ID NO : 2 ou SEQ ID NO : 57 selon l'invention, diffère notamment de SEQ ID NO : 1 ou 2 ou 57 par l'insertion, la suppression ou la substitution d'au moins un nucléotide. Comme on l'entend ici, le pourcentage d'identité entre deux séquences est défini comme le nombre de positions pour lesquelles les bases sont identiques lorsque les séquences sont alignées de manière optimale, divisé par le nombre total de bases de la plus grande des deux séquences. Deux séquences sont dites alignées de manière optimale lorsque le pourcentage d'identité est maximal. Par ailleurs, comme cela apparaitra de manière claire à l'homme du métier, il peut être nécessaire de faire appel à des ajouts de positions lacunaires (des " gaps ") de manière à obtenir un alignement optimal entre les deux séquences.

La présente invention concerne un aptamère modifié, c'est-à-dire un aptamère selon l'invention comprenant au moins un groupement additionnel en plus de l'acide nucléique. Ainsi, l'acide nucléique selon l'invention peut être lié à au moins un groupement additionnel. De manière préférentielle, l'aptamère selon l'invention est constitué de l'acide nucléique selon l'invention et d'au moins un groupement additionnel selon l'invention.

Dans un mode de réalisation, le groupement additionnel de l'invention peut ainsi être un radioisotope, une molécule organique comprenant 100 atomes de carbone au plus, une nanoparticule, une protéine, notamment une glycoprotéine, un glucide, un lipide, ou encore un polynucléotide. Dans un mode de réalisation, le groupement additionnel de l'invention est sélectionné dans le groupe comprenant d'une manière non-limitative : un marqueur détectable, un composé pharmacologique, et un composé susceptible de modifier les caractéristiques pharmacocinétiques d'un acide nucléique auquel il est lié, tel que le polyéthylène glycol (PEG), une structure 3'-CAP- et/ou 5'-CAP-structure et/ou un nucléotide guanosine modifié (tel que 7-methyl-guanosine) en 3'- et/ou en 5' de l'aptamère.

Dans un mode de réalisation, le groupement additionnel de l'invention stabilise l'aptamère de l'invention en augmentant sa demi-vie.

Dans un mode de réalisation, l'aptamère est PEGylé.

Dans un mode de réalisation, le groupement additionnel de l'invention est un énantiomère L et/ou D de l'aptamère de l'invention.

Le marqueur détectable de l'invention comprend mais n'est pas limité à : un fluorophore, par exemple la fluorescéine ou la luciférase; un radioisotope, notamment adapté à la scintigraphie, par exemple 99mTc; une étiquette reconnaissable par un anticorps, par exemple la protéine c-Myc; une étiquette d'affinité, par exemple la biotine; une enzyme, par exemple la peroxydase de raifort.

Selon un autre mode de réalisation, l'aptamère selon l'invention peut être modifié, en totalité ou en partie, notamment pour le rendre résistant à une dégradation hydrolytique, en particulier du fait de l'action de nucléase. De telles modifications sont bien connues de l'homme du métier et recouvrent notamment les modifications de la fonction OH sur le carbone en position 2' du ribose par méthylation, ou la substitution de cette fonction OH par un groupement amino ou par un halogène, notamment par le fluor, ainsi que le recours à un squelette phosphorothioate, ou à des structure de type acide nucléique bloqué (Locked Nucleic Acid, LNA) ou acide nucléique peptidique (Peptide Nucleic Acid, PNA). Ainsi, de manière préférée, l'aptamère selon l'invention est un ARN dont les riboses des nucléotides pyrimidines portent un atome de fluor sur le carbone en position 2', les riboses des nucléotides puriques pouvant être inchangés.

Dans un mode de réalisation de l'invention, l'aptamère de l'invention peut être modifié de façon à faire entrer par vectorisation l'aptamère de l'invention dans une cellule et/ou un tissu et/ou un organe et/ou le compartiment cellulaire cible. Cette modification peut être apportée par l'ajout d'un groupement additionnel pour la vectorisation.

Un but de la vectorisation est de préserver l'aptamère de l'invention, d'augmenter sa solubilité en cas d'hydrophobie trop marquée, de diminuer sa toxicité. La vectorisation peut également avoir pour objectif de contrôler spatialement, temporellement et quantitativement la distribution de l'aptamère de l'invention dans l'organisme. Par exemple, les vecteurs peuvent porter une molécule de ciblage, qui peut être dans un mode de réalisation le ligand d'un récepteur, ou un anticorps contre une protéine surexprimée dans les tissus visés. La vectorisation peut porter aussi sur un transgène dont l'expression sera ciblée par une protéine chimère. Dans un mode de réalisation l'aptamère de l'invention peut être encapsulé avec un agent d'imagerie. L'utilisation de vecteurs sensibles à des stimuli tels que le pH ou la température peut également permettre d'accélérer le relargage ou de le provoquer à l'endroit désiré.

Dans un mode de réalisation, une séquence peptidique ou nucléique ou une molécule d'adressage peut être ajoutée afin d'assurer la vectorisation ou le ciblage de l'aptamère de l'invention vers STAT5. Dans un autre mode de réalisation, une séquence peptidique telle que la séquence TAT peut par exemple être ajoutée pour favoriser l'entrée de l'aptamère de l'invention dans les lymphocytes. Dans un autre mode de réalisation de l'invention, une construction chimérique comprenant un peptide pénétrant les cellules peut être ajouté à un aptamère selon l'invention. Dans un autre mode de réalisation de l'invention, des méthodes d'encapsulation telles que des micelles, polymersomes, liposomes, virus comprenant l'aptamère de l'invention pourront être utilisées. Ces méthodes de vectorisation et leur mise en œuvre sont bien connues de l'homme du métier.

La présente invention concerne également une composition comprenant au moins un aptamère selon la présente invention.

Dans un mode de réalisation, la composition de l'invention comprend au moins SEQ ID NO : 1 ou variant de SEQ ID NO : 1 tel que décrit ci-dessus.

Dans un autre un mode de réalisation, la composition de l'invention comprend au moins SEQ ID NO : 2 ou variant de SEQ ID NO : 2 tel que décrit ci-dessus.

Dans un autre un mode de réalisation, la composition de l'invention comprend au moins SEQ ID NO : 57 ou variant de SEQ ID NO : 57 tel que décrit ci-dessus.

Dans un autre un mode de réalisation, la composition de l'invention comprend SEQ ID NO : 1 ou variant de SEQ ID NO : 1 tel que décrit ci-dessus et SEQ ID NO : 2 ou variant de SEQ ID NO : 2 tel que décrit ci-dessus.

Dans un autre un mode de réalisation, la composition de l'invention comprend SEQ ID NO : 1 ou variant de SEQ ID NO : 1 tel que décrit ci-dessus et SEQ ID NO : 57 ou variant de SEQ ID NO : 57 tel que décrit ci-dessus.

Dans un autre un mode de réalisation, la composition de l'invention comprend SEQ ID NO : 57 ou variant de SEQ ID NO : 57 tel que décrit ci-dessus et SEQ ID NO : 2 ou variant de SEQ ID NO : 2 tel que décrit ci-dessus.

Dans un autre un mode de réalisation, la composition de l'invention comprend (i) SEQ ID NO : 1 ou variant de SEQ ID NO : 1 tel que décrit ci-dessus, et (ii) SEQ ID NO : 2 ou variant de SEQ ID NO : 2 tel que décrit ci-dessus et (iii) SEQ ID NO : 57 ou variant de SEQ ID NO : 57 tel que décrit ci-dessus.

La présente invention concerne également une composition pharmaceutique comprenant au moins la composition de l'invention, en combinaison avec un excipient pharmaceutiquement acceptable.

La présente invention concerne également un médicament comprenant au moins la composition de l'invention.

La présente invention concerne également un aptamère de l'invention, ou une composition, composition pharmaceutique ou médicament de l'invention pour traiter, ou pour son utilisation dans le traitement d'une maladie liée à STAT5, chez un sujet qui en a besoin.

Dans un mode de réalisation, une maladie liée à STAT5 correspond à une surexpression de l'expression génique et/ou protéique de STAT5.

Dans un autre mode de réalisation, une maladie liée à STAT5 correspond à une suractivation de l'activité biologique de STAT5.

Dans un autre mode de réalisation, une maladie liée à STAT5 correspond à une dérégulation de l'activité biologique de STAT5.

Selon un premier mode de réalisation, la maladie liée à STAT5 est un cancer. Des exemples de cancers incluent mais ne sont pas limités à la leucémie, leucémie aigüe, leucémie chronique, leucémie lymphoblastique ou lymphoïde, leucémies myéloblastiques, leucémie aiguë lymphoblastique, leucémie chronique lymphoblastique, leucémie aiguë myéloblastique, leucémie lymphoïde chronique, leucémie myéloïde chronique, leucémie myélomonocytaire juvénile, leucémie prolymphocytaire T de Galton, mycosis fungoïde, à lymphocytes T suppresseurs, tricholeucémie, et leucémies à grands lymphocytes, cancer de la prostate, cancer du sein, cancer métastatique du sein, cancer des poumons, cancer du pancréas, cancer des intestins, cancer de l'utérus, cancer colorectal, un cancer de préférence est une leucémie.

Selon un deuxième mode de réalisation, la maladie liée à STAT5 est un cancer lié à une surexpression et/ou une suractivation de STAT5 et/ou une dérégulation de l'activité biologique de STAT5. Des exemples de ces maladies incluent mais ne sont pas limitées à : la leucémie, la leucémie aigüe, le lymphome anaplasique à grandes cellules, le syndrome Sezary, leucémie lymphoblastique ou lymphoïde, leucémies myéloblastiques, leucémie aiguë lymphoblastique, leucémie chronique lymphoblastique, leucémie aiguë myéloblastique, leucémie myéloïde chronique, leucémie de Burkitt, leucémie myélomonocytaire juvénile, leucémie myélomonocytaire chronique, leucémie prolymphocytaire T de Galton, mycosis fungoïde, tricholeucémie, et leucémies à grands lymphocytes, le cancer de la prostate, le cancer du sein, cancer métastatique du sein; de préférence la maladie liée à STAT5 est une leucémie.

Selon un troisième mode de réalisation, la maladie liée à STAT5 est une leucémie. Des exemples de leucémies incluent mais ne sont pas limitées à : leucémie aigüe, leucémie chronique, leucémie lymphoblastique ou lymphoïde, leucémies myéloblastiques, leucémie aiguë lymphoblastique, leucémie chronique lymphoblastique, leucémie aiguë myéloblastique, leucémie lymphoïde chronique, leucémie myéloïde chronique, leucémie myélomonocytaire juvénile, leucémie prolymphocytaire T de Galton, mycosis fungoïde, tricholeucémie, et leucémies à grands lymphocytes.

Les leucémies aiguës myéloblastiques (ou LAM) comprennent différents stades : LAM 0 : indifférenciée ; LAM 1 : myéloblastique sans différenciation ; LAM 2 : myéloblastique avec différenciation ; LAM 3 : promyélocytaire ; LAM 4 : myélomonocytaire ; LAM 4Eo : myélomonocytaire avec éosinophilie ; LAM 5 : monoblastique (sans différenciation : M5a, avec différenciation : M5b) ; LAM 6 : érythroblastique ou érythroleucémie ; LAM 7 : mégacaryoblastique. De même, les leucémies aiguës lymphoblastiques (ou LAL) comprennent les stades suivants : LAL 1 ; LAL 2 ; LAL 3 ou lymphome de Burkitt ; le type L3 correspondant toujours à des proliférations B. Les types L1 et L2 peuvent correspondent à des proliférations pré-B ou pro-pré-B, avec des degrés variables de différenciation, ou à des proliférations T.

Dans le cas où cette classification changerait, l'homme du métier saurait à quel type de maladie la nouvelle classification correspondrait.

Dans un mode de réalisation, la maladie liée à STAT5 ne comprend pas la leucémie lymphoïde chronique ; la tricholeucémie ; les leucémies à grands lymphocytes ; ou les leucémies prolymphocytaires.

Selon un mode de réalisation de l'invention, la composition, la composition pharmaceutique ou le médicament de l'invention sont adaptés à une administration orale. Au sens de la présente invention, on entend par « administration orale » une administration dans la cavité buccale, suivie par l'ingestion d'un aptamère selon l'invention, qui rejoint la circulation systémique à la suite de son absorption intestinale.

Selon un mode de réalisation de l'invention, la composition, la composition pharmaceutique ou le médicament de l'invention est sous une forme solide. Des exemples de formulations solides adaptées à une administration orale incluent, mais ne sont pas limités à, des granules, une poudre, une gélule, un comprimé, une pommade, un gel, une poudre à dissoudre, une pâte, une gomme à mâcher, une capsule souple ou une capsule molle.

Des exemples de véhicules, diluants ou excipients solides incluent, mais ne sont pas limités au glucose, fructose, sucrose, maltose, dextrine jaune, dextrine blanche, maltodextrine, cellulose microcristalline, stéarate de calcium, stéarate de magnésium, sorbitol, sirop de glucose, lactose, acide citrique, acide tartrique, acide malique, acide succinique, acide lactique, acide L-ascorbique, alpha-tocophérol, glycérol, propylène glycol, sucroester, esters poly glycériques d'acides gras, sucroglycérides, mono-, di- et triglycérides béhénate, carraghénanes, gomme arabique, caséine, gélatine, pectine, agar, nicotinamide, acides aminés, sels de calcium, pigments.

Selon un autre mode de réalisation de l'invention, la composition, la composition pharmaceutique ou le médicament de l'invention est sous forme liquide. Des exemples de formulations liquides adaptées à une administration orale incluent, mais ne sont pas limités à, une solution, une suspension, une émulsion (émulsion huile dans eau, eau dans huile, anhydre, solide ou microémulsions), un spray, un inhalateur, une ampoule comprenant la composition, la composition pharmaceutique ou le médicament de l'invention, une poudre à dissoudre, une boisson ou un sirop.

Des exemples de véhicules liquides incluent, mais ne sont pas limités à, de l'eau distillée, une solution saline, une solution aqueuse de glucose, de l'alcool par exemple de l'éthanol, du propylène glycol, et du polyéthylène glycol ; et des véhicules huileux tels que des huiles végétales et animales, de la paraffine, ou de la cire.

Des exemples d'antioxygènes incluent mais ne sont pas limités à du tocophérol, du butylhydroxytoluène (BHT), du butylhydroxyanisol (BHA), des antioxydants naturels comme la vitamine E, de l'extrait de romarin, du gallate 5 de propyle.

Des exemples de conservateurs antimicrobiens incluent mais ne sont pas limités à du méthylparabène, du propylparaben, du sorbate de potassium, du benzoate de sodium, de l'acide benzoïque.

Des exemples d'antiagglomérants incluent mais ne sont pas limités à du dioxyde de silicium.

Des exemples de surfactants incluent mais ne sont pas limités à des surfactants anioniques, cationiques, ou non-ioniques tels que le palmitate d'ascorbyl, les polysorbates, les polyéthylènes glycols.

Des exemples d'agents stabilisateurs du pH ou des tampons incluent mais ne sont pas limités à acide citrique-citrate de sodium, acide phosphorique-phosphate de sodium, acide acétique-acétate de sodium.

Dans un autre mode de réalisation de l'invention, la composition, la composition pharmaceutique ou le médicament selon l'invention est formulée sous forme de comprimés à libération contrôlée, utilisant des revêtements à base de polymères permettant la libération contrôlée grâce à des techniques bien connues de l'homme du métier telles que la micro-encapsulation ou des systèmes de véhicule colloïdal. Des exemples d'agents d'encapsulation incluent, mais ne sont pas limités à, de l'amidon, des protéines de source animale comme, par exemple, la gélatine, des protéines de source végétale, de la caséine, de la pectine, de l'alginate, de l'agar, des maltodextrines, des sulfonates de lignine, des dérivés cellulosiques (éthylcellulose, méthylcellulose, hydroxypropylcellulose, hydroxypropylméthylcellulose, carboxyméthylcellulose), des sucres, sorbitols, gommes....

Selon un mode de réalisation de l'invention, la composition, la composition pharmaceutique ou le médicament de l'invention sont adaptés à une administration par injection, telle que, par exemple, par injection intraveineuse, intrathécale, intradermale, intramusculaire ou épidurale.

Des exemples de formulations adaptées à une administration par injection incluent, mais ne sont pas limités à, une solution injectable et une émulsion injectable (telle que, par exemple, une émulsion huile dans eau, une émulsion eau dans huile, une émulsion anhydre, une émulsion solide ou une microémulsion) comprenant les agonistes selon l'invention.

Selon un mode de réalisation de l'invention, la composition, la composition pharmaceutique ou le médicament de l'invention sont adaptés à une administration topique, de préférence à une administration transcutanée. Par "administration transcutanée", on entend l'administration d'un composé sur la peau, suivie de son absorption dans la circulation sanguine systémique à travers les tissus cutanés adjacents.

Des exemples de formulations adaptées à une administration topique, de préférence transcutanée incluent, sans y être limités, une pommade, une pâte, un onguent, un gel, une crème ou un patch transdermique.

Selon un mode de réalisation de l'invention, l'aptamère selon l'invention, ou la composition, la composition pharmaceutique ou le médicament de l'invention est formulé sous la forme d'un dosage unitaire. Des exemples de dosages unitaires incluent, mais ne sont pas limités à, un comprimé, une gélule, une ampoule ou une solution injectable.

La présente concerne également un dosage unitaire comprenant l'aptamère selon l'invention.

Selon un mode de réalisation de l'invention, la quantité de l'aptamère selon l'invention administrée au sujet varie d'environ 1 µg/kg de masse corporelle à environ 10 mg/kg de masse corporelle, de préférence d'environ 10 µg/kg à environ 5 mg/kg, plus préférentiellement d'environ 50 µg/kg à environ 1 mg/kg.

Selon un autre mode de réalisation, l'homme du métier pourra adapter l'administration de l'aptamère de l'invention en fonction du type de cancer, de la sévérité de la maladie, de l'âge, du sexe du sujet.

Dans la présente invention, le terme "sujet" désigne un animal, de préférence un mammifère, plus préférentiellement un être humain. Selon un mode de réalisation de l'invention, le sujet est un homme. Selon un autre mode de réalisation de l'invention, le sujet est une femme. Selon un mode de réalisation de l'invention, le sujet est un adulte. Selon un autre mode de réalisation de l'invention, le sujet est un enfant. Selon un autre mode de réalisation de l'invention, le sujet est un adolescent. Dans la présente invention, le sujet enfant est âgé de 0, 1, 2, 3, 4, 5, 6, 7, 8, 9, 10, 11, 12, 13 ans. Dans la présente invention, le sujet adolescent est âgé de 14, 15, 16, 17, 18, 19 ans. Dans la présente invention, le sujet adulte est âgé d'au moins 20 ans.

Dans un premier mode de réalisation, ledit sujet souffre d'une maladie liée à STAT5, de préférence d'un cancer, plus préférentiellement est diagnostiqué comme souffrant d'une maladie liée à STAT5, de préférence d'un cancer.

Dans un deuxième mode de réalisation, ledit sujet est à risque de développer une maladie liée à STAT5, de préférence un cancer.

Selon un troisième mode de réalisation, le sujet présente des prédispositions non génétiques à une maladie liée à STAT5, de préférence à un cancer.

Des facteurs de risques induisant des cancers incluent mais ne sont pas limités à : antécédent de radiothérapie ou de chimiothérapie pour un autre cancer ; exposition à la radioactivité ; exposition *in utero* aux rayons X ; exposition à certains produits chimiques (benzène, les hydrocarbures aromatiques) ou à certains engrais ; exposition (y compris *in utero* à de faibles doses) à certains pesticides ; selon une méta étude faite sur 31 études épidémiologiques faites entre 1950 et 2009, l'exposition de la mère lors de son travail durant la grossesse double le risque de déclaration d'une leucémie chez l'enfant (augmentation de 40 % chez les agricultrices qui semblent les plus exposées). Ce risque de leucémie infantile augmente le plus avec l'exposition à des insecticides et des herbicides (+ 2,7 et + 3,6 respectivement) ; certaines anomalies génétiques comme la trisomie 21 ; certaines maladies comme le rachitisme, certaines infections et le cancer de la moelle osseuse ; maladies hématologiques myéloprolifératives : polyglobulie essentielle ou maladie de Vaquez, myélofibrose (fibroblastes en prolifération), anémie aplasique (beaucoup ne sont en fait que des leucémies) tandis qu'une leucémie chronique se transforme souvent en leucémie aiguë ; exposition aux émanations de certains objets de décoration (exemple: composés organiques volatiles totaux et méthanal) ; ou toute cause encore inconnue à ce jour (9 cas sur 10).

Selon un quatrième mode de réalisation, le sujet présente des prédispositions génétiques à une maladie liée à STAT5, de préférence à un cancer, plus préférentiellement à une leucémie.

Des exemples de maladies diagnostiquées chez un sujet augmentent le risque de développement de maladies liées à STAT5. Elles incluent de manière non-limitative : les syndromes myélodysplasiques, la maladie de Fanconi, la trisomie 21, les thrombopénies familiales, T-cell Leukemia Virus-1.

Des exemples de prédispositions génétiques à une leucémie incluent mais ne sont pas limitées à : des mutations dans les gènes *FANCA, FANCB, FANCC, FANCD1* (également connu sous le nom *BRCA2* ce gène est impliqué dans les cancers du sein familiaux), *FANCD2, FANCE, FANCF, FANCG, FANCI, FANCJ, FANCL, FANCM* et *FANCN,* déficit en p53, mutations dans le gène GATA2, RUNX1, ou la présence d'une forme singulière du gène PRDM9 dans les cellules sexuelles des parents.

La présente invention concerne également une composition, une composition pharmaceutique ou un médicament destiné à être administré en combinaison avec d'autres agents anti-cancéreux, (en particulier des agents anti-leucémiques), agents anti-métastatiques, des agents anti-foliques, des agents anti-métabolites tels que par exemple des agents antipuriques, des agents antipyrimidiques, des agents alkylants tels que par exemple de la moutarde azotée, la nitroso-urée, l'organoplatine, l'éthylène imine, l'amide imidazole, des agents intercalants tels que par exemple des dérivés de la camptothécine, l'anthracycline, des agents agissant sur le fuseau mitotique tels que par exemple : des agents vinca-alcaloïde, des taxoïdes, les inhibiteurs des tyrosines kinases tels que par exemple Dasatinib, Erlotinib, Imatinib, Sorafénib, Sunitinib, des agents anti-angiogéniques, des agents différenciants tels que l'acide tout-trans rétinoïque et sels d'arsenic, ou un mélange de ceux-ci.

Dans un mode de réalisation, la composition, la composition pharmaceutique ou le médicament de l'invention est destiné à être administré avant, pendant, et/ou après une chimiothérapie.

Dans une autre mode de réalisation, la composition, la composition pharmaceutique ou le médicament de l'invention est destiné à être administré avant, pendant, et/ou après une radiothérapie.

Dans une autre mode de réalisation, la composition, la composition pharmaceutique ou le médicament de l'invention est destiné à être administré avant, et/ou après une allogreffe. La présente invention concerne également un aptamère de l'invention, ou une composition, composition pharmaceutique ou médicament de l'invention destiné à être administré en combinaison avec d'autres agents anti-cancéreux, (en particulier des agents anti-leucémiques), agents anti-métastatiques, des agents anti-foliques, des agents anti-métabolites tels que par exemple des agents antipuriques, des agents antipyrimidiques, des agents alkylants tels que par exemple de la moutarde azotée, la nitroso-urée, l'organoplatine, l'éthylène imine, l'amide imidazole, des agents intercalants tels que par exemple des dérivés de la camptothécine, l'anthracycline, des agents agissant sur le fuseau mitotique tels que par exemple : des agents vinca-alcaloïde, des taxoïdes, les inhibiteurs des tyrosines kinases tels que par exemple Dasatinib, Erlotinib, Imatinib, Sorafénib, Sunitinib, des agents anti-angiogéniques, des agents différenciants tels que l'acide tout-trans rétinoïque et sels d'arsenic, ou un mélange de ceux-ci, pour traiter, ou pour son utilisation dans le traitement d'une maladie liée à STAT5, chez un sujet qui en a besoin.

La présente invention concerne également une méthode de traitement d'une maladie liée à STAT5 chez un sujet, ladite méthode comprenant l'administration audit sujet d'une quantité efficace de l'aptamère de l'invention, ou de la composition, composition pharmaceutique ou médicament de l'invention.

Dans un mode de réalisation, la méthode de l'invention inhibe et/ou stoppe et/ou prévient la surexpression de STAT5 et/ou la suractivation de STAT5 et/ou l'hyperphosphorylation de STAT5 et/ou la fixation de STAT5 sur l'ADN. L'homme du métier sait comment mesurer par immunochimie le niveau de phosphorylation de protéines.

Dans un autre mode de réalisation, la méthode de l'invention inhibe et/ou stoppe et/ou prévient la prolifération de cellules cancéreuses. L'homme du métier peut mesurer la prolifération de cellules par des méthodes bien connues de l'état de l'art, par exemple par comptage.

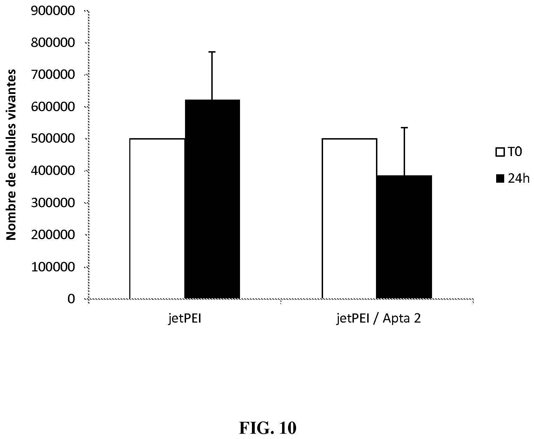

Dans un autre mode de réalisation, la méthode de l'invention induit et/ou rétablit les mécanismes de l'apoptose des cellules cancéreuses. L'homme du métier peut mesurer l'effet d'un composé sur les mécanismes de l'apoptose à l'aide de tests tels que les tests MTT, les tests de la LDH, le test de Tunel, des kits permettant de mesurer spécifiquement différents marqueurs de l'apoptose comme dans la figure 10 de la présente invention.

Dans un autre mode de réalisation, la méthode de l'invention inhibe la voie de signalisation STAT/Jak. L'homme du métier sait comment mesurer par immunochimie, ELISA, la modulation de la voie de signalisation STAT/Jak.

Un autre objet de l'invention est un procédé de détection *in vitro* de STAT5 dans un échantillon biologique comprenant :
a. la mise en contact d'au moins un aptamère selon l'invention avec un échantillon de cellules d'un sujet préalablement prélevé,
b. la détermination de la quantité dudit aptamère lié audit échantillon.

Dans un mode de réalisation, le procédé de détection peut permettre de suivre l'évolution de la maladie liée à STAT5.

Dans un mode de réalisation, ledit procédé de diagnostic *in vitro* d'une maladie liée à STAT5 dans un échantillon de cellules comprend :
a. la mise en contact d'au moins un aptamère selon l'invention avec un échantillon de cellules d'un sujet préalablement prélevé,
b. la détermination de la quantité dudit aptamère lié audit échantillon,
c. la comparaison de ladite quantité à un contrôle référent,
dans lequel une augmentation de la détection dudit aptamère lié à l'échantillon correspond à la mise en évidence d'une maladie de l'invention.

Le terme échantillon biologique utilisé dans la présente invention a été préalablement prélevé comprend mais n'est pas limité à un échantillon de sang, de sérum, plasma, cellules provenant de tissus ou d'organes, liquide céphalo rachidien, urine, ascite.

Dans un mode de réalisation, le contrôle référent tel qu'utilisé dans la présente invention correspond à un échantillon biologique dont le niveau d'expression STAT5 est connu de l'homme du métier. Il peut s'agir de comparer le niveau d'expression STAT5 provenant de l'échantillon biologique d'un sujet avec le niveau d'expression STAT5 provenant de l'échantillon biologique d'un sujet ne présentant pas de maladie liée à STAT5 ou d'un sujet présentant une maladie liée à STAT5.

### BRÈVE DESCRIPTION DES FIGURES

**Figure 1** représente la séparation sur PAGE-urée 7 M (gel 15% PAGE-urée 7 M) des brins sens et antisens des aptamères sélectionnés et amplifiés par PCR. Le produit de la PCR (100 µL + 25 µL urée 5X) est déposé dans un puits unique et la migration en tampon TBE se fait 55 minutes à 200 V (A) Révélation par mesure de la fluorescence. (B) Révélation au bleu de méthylène.
**Figure 2** représente une évolution apparemment très dispersée de la quantité d'aptamère récupérée, mais la courbe de tendance indique qu'il y a un enrichissement d'un facteur 1,33.
**Figure 3** représente la structure de Apta 1 d'après le logiciel mfold.
**Figure 4** représente la structure de Apta 2 d'après le logiciel mfold.
**Figure 5** représente la structure de Apta 3 d'après le logiciel mfold.
**Figure 6** représente la mise en évidence de l'interaction entre STAT5 et Aptal. Les résultats du pool down du Western Blot sont présentés. MW : Marqueur de taille (Dual color - BIORAD). Et : Extrait protéines totales. NR : Fraction non retenue. W1, W2, W3, W4 : Fractions de lavages successives. El, E2 : Fractions d'élution successives.
**Figure 7** représente la croissance des cellules KU-812 en l'absence (JetPEI) ou en présence (Aptamèrel/JetPEI) d'Apta1. Mesure du nombre de cellules à t=0 (blanc), t=24h (noir). Moyenne obtenue sur 5 expériences.
**Figure 8** représente la mise en évidence de la mortalité cellulaire des cellules KU-812 en l'absence (JetPEI) ou en présence (Aptamèrel/JetPEI) d'Apta1, à raison de 6 µg d'ADN/500 000 cellules. Les cellules sont colorées au bleu Trypan après 24h de culture et comptées sur cellule de Malassez.
**Figure 9** représente la révélation des membranes du Kit Human Apoptosis Antibody Array (R&D Systems). L'analyse est faite à partir de l'application Image Studio qui mesure l'intensité des spots. L'intensité du signal est dépendante de l'expression de la protéine cible dans l'extrait protéique. Les histogrammes correspondent à la différence d'expression entre cellules non traitées et cellules transfectées avec Aptal/JetPel pendant 48h (300 000 cellules/mL).
**Figure 10** représente la croissance des cellules KU-812. JetPEI: Cellules transfectées par JetPEI, JetPEI/Apta2 : Cellules traitées par JetPEI/Apta2. Apta2. Mesure du nombre de cellules à t=0 (blanc), t=24h (noir). Moyenne obtenue sur 2 expériences.
**Figure 11** représente la croissance des cellules KU-812 transfectées toutes les 24 heures par 6 µg d'Apta2.
**Figure 12** représente la viabilité cellulaire des cellules KU-812 transfectées par différentes concentrations d'Apta2 : 50 nM ; 150 nM ; 300 nM en fonction du temps (6h ; 12h ; 24h).
**Figure 13** représente l'effet apoptotique de 150 nM d'Apta2 transfecté dans les cellules KU-812.

### EXEMPLES

La présente invention se comprendra mieux à la lecture des exemples suivants.

### Matériel et Méthodes

### Méthode pour la production de protéines recombinantes STAT5 :

### Digestion

L'extraction du plasmide pTAT/HA est réalisée à partir de pré-cultures bactériennes de 24 heures avec le kit QiaPrep Spin Mini-Prep (Qiagen).

Le gène stat5B est digéré par EcoRI et KpnI dans les conditions suivantes : plasmide pTAT/HA/stat5B 1 µg, KpnI 1 unité, EcoRI 1 unité, NEB-1 1X, BSA 1X, H₂O qsp 50 µL. Le mélange est incubé 1 heures à 37°C

### Ligation du gène stat5B dans le vecteur pRSETB

Le vecteur pRSETB est digéré par KpnI et EcoRI dans les mêmes conditions que le gène stat5B.

pRSET est déphosphorylé par action d'une phosphatase alkaline, la Rapid Alkaline Phosphatase (Roche) dans les conditions suivantes : ADN plasmidique 1µg (maximum), Rapid Alkaline phosphatase buffer 1X, Rapid Alkaline phosphatase 1 unité, H₂O qsp 20 µL. Le mélange est incubé 10 minutes à 37°C puis 2 minutes à 75°C.

La ligation est réalisée dans les conditions suivantes : ADN plasmidique 1 µg (maximum), insert 1µg (maximum), ligation buffer 1X, T4 DNA ligase (Roche) 3 unités, H₂O qsp 30 µL. Le mélange est incubé à 16°C pendant 12 heures.

### Transformation bactérienne par la construction pRSETB/stat5B

Les cellules *E. coli* sont préalablement rendues compétentes, sont transformées par la construction plasmidique pRSETB/stat5B.

100 ng d'ADN sont ajoutés à 100 µL de cellules compétentes tout juste décongelées et conservées sur la glace. Le tout est incubé 20 minutes sur glace, puis 45 secondes à 42°C. Le mélange est ensuite refroidi 2 minutes sur glace. 900 µL de milieu SOC (1 % tryptone ; 1% extrait de levure ; 10 mM NaCl ; 2,5 mM KCl ; 10 mM MgCl₂ ; 10 mM MgSO₄ ; 20 mM glucose) préchauffé à 37°C sont ajoutés et le mélange est incubé 1h30 à 37°C sous légère agitation. 100 µL sont étalés sur des boites de Petri contenant du milieu LB-agar/ampicilline solide (1 % tryptone ; 1% extrait de levure ; 10 mM NaCl ; 2% agar ; ampicilline 100 µg/mL). Les clones poussent à 37°C pendant la nuit. Les clones sont mis en culture et stockés à -80°C dans 15% glycérol.

### Production, purification et renaturation des protéines recombinantes STAT5B

10 µL de stock glycérolé de cellules transformées par pRSETB/STAT5B sont ajoutés à 10 mL de milieu LB/ampicilline (5 g NaCl, 5 g d'extrait de levure, 10 g de Tryptone ; 100 µg/mL d'ampicilline). Les cellules sont cultivées 12 heures à 37°C sous agitation (180 rpm- INFORS HT).

La culture est diluée de sorte à ce que l'absorbance à 600 nm soit de 0,1 puis remise à 37°C sous agitation (180 rpm - INFORS HT).

L'induction de la production protéique est réalisée lorsque la culture atteint une absorbance à 600 nm de 0,4 à 0,6. Les protéines sont ensuite extraites de cellules.

Les protéines recombinantes sont purifiées sur résine Ni-NTA Agarose (Qiagen). Les protéines recombinantes STAT5B sont renaturées par dialyses successives

### Mesure de la quantité de protéines (test BCA)

Une gamme étalon de concentration d'Albumine de Sérum Bovin (BSA) est préparée (0 ; 25 ; 50 ; 75 ; 100 ; 150 ; 200 ; 300 et 500 µg/mL). Le réactif BCA est composé d'une solution A d'acide bicinchoninique et d'une solution B à 4% de sulfate de cuivre (A/B : 50/1). 10 µL de la gamme et de chaque échantillon sont déposés en duplicates dans une plaque 96 puits. Le réactif BCA est ajouté à raison de 200 µL/puits. La plaque est incubée 30 minutes à 37°C, puis l'absorbance à 560 nm est mesurée. L'intensité de la coloration est proportionnelle à la concentration massique des protéines.

### Méthode SELEX d'isolation d'aptamères

La banque aléatoire d'oligonucléotides, inspirée de Stoltenburg et al. (2005. Anal Bioanal Chem. 383(1):83-91) a été synthétisée sous la forme suivante (Eurogentec) :
5' ATACCAGCTTATTCAATT N60 AGATAGTAAGTGCAATCT 3' où N est aléatoirement A, T, G ou C (SEQ ID NO : 84).

### Capture et élution

Les protéines STAT5B purifiées sont capturées sur des billes Dynabeads® His-Tag Isolation & Pulldown (Dynal) par incubation (1 heure à température ambiante) de 2 mg de billes avec 150 à 200 µg de protéines STAT5B. La banque d'oligonucléotides est ajoutée à raison de 3 nmol (tour 1) ou 200 pmol (tour 2 à n). Le tampon liaison/lavage est ajouté qsp 700 µL aux billes qui sont incubées toute la nuit sous agitation à 4°C.

L'élution des aptamères fixés à STAT5B est réalisée en ajoutant 200 µL de tampon d'élution (10 mM EDTA ; 40 mM Tris-HCl ; 3,5 mM urée ; 0,02 % Tween-20) et en incubant 7 minutes à 80°C. L'élution est répétée 2 fois.

### Amplification et séparation des aptamères

Les aptamères sont amplifiés par PCR en utilisant les amorces suivantes, l'une (sens) fluorescente, l'autre (antisens) alourdie :
Sens 5'-Fluo-ATACCAGCTTATTCAATT-3' (SEQ ID NO : 9)
Anti-sens 5'-Poly-dA20-AGATTGCACTTACTATCT-3' (SEQ ID NO : 10)

Le mélange de PCR est le suivant : aptamère (totalité récupérée, soit environ 3 pmol) ; ADN polymérase (Vent polymerase - New England Biolabs) 2 unités ; amorces sens 10 µM ; amorces anti-sens 10 µM ; tampon de PCR 1X ; dNTP 25 mM ; H₂O qsp 50 µL.

Les cycles de température sont réalisés dans un thermocycleur Biorad C1 000TM Thermal Cycler selon le programme suivant (**Tableau 1**) :

**Tableau 1 : Cycles PCR pour l'amplification des aptamères sélectionnés.**

| | Cycles | Temps | Température |
|---|---|---|---|
| Dénaturation initiale | 1 | 5 min | 94°C |
| Dénaturation | | 1 min | 94°C |
| Hybridation | 30 | 1 min | 47°C |
| Elongation | | 1 min | 72°C |
| Fin de l'élongation | 1 | 1 min | 72°C |
| Fin de réaction | 1 | ∞ | 4°C |

Les brins sens et antisens sont séparés par électrophorèse PAGE-urée 7 M après purification sur colonne QIAquick PCR Purification (Qiagen). Les résultats sont révélés aux UV.

Les brins sens sont ensuite purifiés à partir de la bande détectée aux UV. La bande est découpée et plongée dans le tampon de diffusion du Kit NucleoSpin Gel (Macherey Nagel), à raison de 200 µL pour 100 ng de gel. Le morceau de gel est broyé et incubé toute la nuit à 37°C, puis centrifugé 5 minutes à 13000 g. 2 volumes de tampon NTC (fourni par le kit) sont ajoutés au surnageant. Les aptamères sont purifiés avec le kit Clean-up (Macherey-Nagel), en répétant l'étape d'élution 5 fois.

### Séquençage des aptamères sélectionnés

L'analyse des aptamères sélectionnés est réalisée par clonage des séquences retenues dans un système d'expression adapté. Le clonage est effectué par l'intermédiaire de vecteur d'expression pGEMT®. Le séquençage est réalisé à partir d'amorces standards T7.

### Mise en évidence de l'interaction aptamère-STAT5

### Extraction des protéines STAT5 cellulaires activées.

Deux sources cellulaires de STAT5 activées ont été exploitées :
- la lignée murine pro-lymphocytaire Ba/F3 transformée par la forme oncogénique de STAT5B, appelée STAT5B 1^{∗}6,
- la lignée humaine myéloïde KU-812, établie à partir d'un patient atteint de leucémie myéloïde chronique (avec chromosome Ph).

Les expériences suivantes sont réalisées à froid (sur glace et centrifugations à 4°C).

10 millions de cellules sont centrifugées 2 minutes à 260 g. Le culot de cellules est lavé par du PBS froid (par 5 mL, puis par 1 mL) puis centrifugé de la même façon. Les cellules sont reprises dans 50 µL de tampon EC (Hépès 20 mM pH7,9 ; KCl 10 mM ; EDTA 1 mM ; NP40-Tergitol® 0,2% ; glycérol 10% auxquels sont ajoutés extemporanément : phenylmethylsulfonyl fluoride 2 mM ; dithiothreitol 1 mM ; orthovanadate de sodium 1 mM ; Complete EDTA Free - Roche), incubées 5 minutes dans la glace, puis centrifugées 2 minutes à 12000 g. Le surnageant contient les protéiques STAT5 du cytoplasme.

Le culot est ensuite repris par 50 µL de tampon EN (Hépès 20 mM pH7,9 ; KC110 mM ; EDTA 1 mM ; NaCl 400 mM ; glycérol 20% auxquels sont ajoutés extemporanément : phenylmethylsulfonyl fluoride 2 mM ; dithiothreitol 1 mM ; orthovanadate de sodium 1 mM ; Complete EDTA Free - Roche), incubé 30 minutes dans la glace en agitant de temps en temps le tube. Le mélange est ensuite centrifugé 2 minutes à 16100 g. Le surnageant contient les protéines STAT5 nucléaires.

### Pull Down

200 pmol d'aptamères biotinylés sont incubés avec 200 µg de protéines des extraits cytoplasmiques et nucléaires pendant 2 heures sur glace dans le tampon de liaison/lavage (5X : Tris pH 7,5 50 mM ; NaCl 50 mM ; EDTA 5 mM ; PMSF 2,5 mM ; glycérol 25% ; NP40-Tergitol® 0,5%). Le mélange est ensuite mis en contact avec 100 µL de billes streptavidine (Dynal) pendant 30 minutes sous agitation à 4°C. Le tout est lavé 3 fois avec le tampon de lavage. Les protéines sont ensuite éluées en ajoutant 40 µL de tampon d'élution (Tris 0,5 M 250 mM pH 6,8 ; glycérol 25%, SDS 8%, β-mercaptoéthanol 20% ; H₂O qsp 10 mL) et en chauffant 5 minutes à 90°C.

### Électrophorèse et Western Blot

Les protéines sont analysées par Western Blot après migration électrophorétique sur gel SDS-PAGE.

Les protéines STAT5 activées sont détectées en exploitant 2 anticorps (aux concentrations indiquées par le fournisseur) : anticorps anti-STAT5 (C-17 Santa Cruz) produit chez le lapin ; anticorps anti-PhosphoSTAT5 (Y694) produit chez le lapin (Cell Signaling).

La reconnaissance spécifique de ces anticorps est ensuite révélée par un anticorps secondaire (anti IgG de lapin) marqué à la peroxydase (Sigma-Aldrich) dilué au 5000^{ème}.

### Mesure de l'effet des aptamères sur les lignées cellulaires

Les cellules des lignées leucémiques sont maintenues en culture en milieu RPMI, 10% SVF, 1% glutamine, 1% pénicilline/streptomycine par incubation à 37°C sous atmosphère humide 5% CO₂. La transfection des aptamères se fait sur 500 000 cellules. 6 µg d'aptamères biotinylés et 12 µL de JetPEI (- Polyplus transfection™) sont respectivement dilués dans 100 µL de NaCl 150 mM. Les 2 solutions sont mélangées et versées sur les cellules à traiter. Les cellules sont incubées 24 heures.

Pour estimer la viabilité cellulaire, les cellules vivantes sont comptées sur cellules de Malassez selon le test d'exclusion au Bleu Trypan.

### Mesure de l'effet des aptamères sur l'expression génique

L'effet des aptamères sur l'expression de tout un ensemble de gènes a été étudié sur les cellules de lignée leucémique préalablement transfectées. Les protéines sont extraites est analysé par Western Blot par le Kit Human Apoptosis Antibody Array (R&D Systems).

### Étude de l'apoptose

La technique TUNEL (*Terminal deoxynucleotidyl transferase dUTP nick end labeling*) est basée sur la présence dans les cellules apoptotiques de fragments d'ADN double-brin de faible poids moléculaire (mono- ou oligonucléosomes) mais aussi des fragments simple-brin de haut poids moléculaire. Ceux-là résultent de la fragmentation de l'ADN au cours du processus apoptotique. Le principe de la technique TUNEL consiste à marquer les extrémités de ces fragments par l'utilisation de l'enzyme désoxynucléotidyl-transférase terminale qui catalyse l'ajout des nucléotides marqués à la fluorescéine aux extrémités 3'OH libres.

### Exemple 1 : Ingénierie de la protéine STAT5

La protéine STAT5B murine (SEQ ID NO : 8) est générée à partir d'un vecteur pTAT-HA/statB5 qui a été sous-cloné par des étapes de digestion, ligation, transformation bactérienne. Etant donné que l'art antérieur a montré une meilleure production de la protéine recombinante STAT5B par rapport à la protéine recombinante STAT5A murine (SEQ ID NO : 7), il a été décidé de produire STAT5B pour produire des aptamères spécifiques de STAT5. Après séquençage, les constructions vectorielles ont été introduites dans un système d'expression procaryote dans le but de produire la protéine recombinante. Comme la plupart des protéines recombinantes, les protéines STAT5B ont tendance à s'agréger et à former des corps d'inclusion dans le cytoplasme. Les protéines ont donc été extraites à partir de ces corps d'inclusions grâce à des tampons dénaturants. Les protéines sont ensuite purifiées sur résine Ni-NTA grâce à l'étiquette 6-His fusionnée aux protéines produites. Cette étape est suivie d'une étape de renaturation telle que décrite dans le matériel et méthode.

Le bilan de production/purification de la protéine recombinante STAT5B est effectué sur gel SDS PAGE 10%. L'identification de la bande correspondant à Stat5b est validée par un Western Blot. La protéine STAT5B a bien été extraite et purifiée. Son degré de pureté est suffisant pour qu'elle serve de cible à la procédure SELEX.

La saturation des billes est optimisée. Les billes sont saturées lorsqu'une quantité mesurable (> 25 µg/mL, limite de sensibilité du test BCA) est présente dans la fraction non retenue.

### Exemple 2 : Isolation, Caractérisation d'aptamères de STAT5

Pour sélectionner des inhibiteurs spécifiques des protéines recombinantes STAT5B précédemment produites la stratégie mise en œuvre a reposé sur la sélection d'aptamères. Les aptamères sont des oligonucléotides synthétiques d'ADN ou d'ARN capables de s'organiser en structures tridimensionnelles complexes. La sélection d'aptamères revient donc à identifier à partir d'une banque d'acides nucléiques les structures les plus complémentaires de la cible, autrement dit celles engageant le plus d'interaction stables.

Les aptamères se caractérisent également par leurs propriétés. Il s'agit de molécules de petite taille composées d'acides nucléiques les rendant faiblement immunogènes. Ils montrent une affinité élevée et une spécificité remarquable pour leur cible et de sélectivité. Les aptamères peuvent être générés contre des cibles de nature très diverse allant des petites molécules organiques aux cellules intactes en passant par les peptides et autres protéines.

Les aptamères sont isolés *in vitro* par une méthode de sélection itérative appelée méthode SELEX. La méthode SELEX est initiée à partir d'une banque d'acides nucléiques dont les séquences flanquantes sont connues. Les banques classiquement exploitées contiennent entre 10¹³ et 10¹⁵ séquences différentes. Cette méthode permet de sélectionner des ligands structurés de forte affinité et spécificité pour la protéine étudiée.

Cette technique consiste à mettre en contact une large banque randomisée d'oligonucléotides avec la protéine recombinante STAT5B précédemment produite - isolée - purifiée et refoldée. Après incubation les oligonucléotides n'ayant pas fixé la protéine cible sont éliminés par lavages alors que les séquences d'ADN complémentaires sont éluées et amplifiées par PCR. Cette étape permet de générer un nouveau pool d'ADN double brin. Une étape de purification sur PAGE est nécessaire afin de séparer les brins complémentaires (dont l'une des séquences est homologue aux aptamères et l'autre complémentaire) et ainsi constituer une nouvelle banque enrichie en séquence présentant toute une affinité +/- forte pour la protéine STAT.

Le second cycle de SELEX consiste à incuber cette nouvelle banque enrichie en ADN avec la protéine cible afin d'éliminer progressivement les séquences de moins en moins affines.

La méthode SELEX repose donc sur la répétition des étapes de lavages / élution / amplification et purification.

Dans un premier temps il a donc fallu vérifier la diversité de la banque d'ADN afin de s'assurer de l'hétérogénéité du mélange. Pour se faire, la banque a été clonée dans le système d'expression pGEMT et la construction plasmidique introduite dans les bactéries *E. coli* compétentes. Les différents clones obtenus ont été étudiés par PCR sur colonies et les produits de PCR envoyés à séquencer. Les résultats concernant les 20 clones séquencés permettent de souligner l'absence de redondance séquentielle et de vérifier que les oligonucléotides de la banque initiale sont conformes à la demande faite auprès du prestataire de service (Eurogentec). Après plusieurs cycles, et grâce à la pression de sélection seulement une ou quelques séquences d'ADN sont retenues. Une fois sélectionnés les oligonucléotides sont séquencés et étudiés afin de déterminer leur pouvoir inhibiteur envers les facteurs de transcription STAT5.

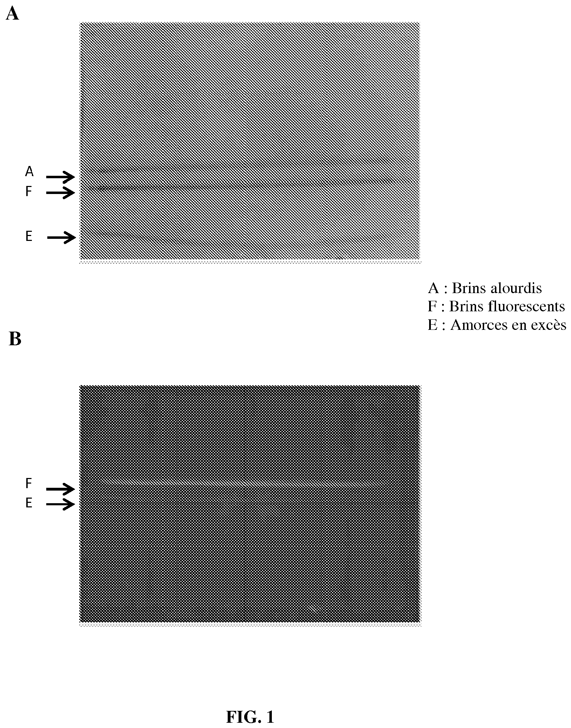

Après mise en contact de la banque et de la protéine STAT5B (SEQ ID NO : 8), lavages et élution, les aptamères sélectionnés sont amplifiés par PCR. Le résultat de la PCR est déposé sur gel PAGE-urée 7M pour être caractérisé (**Figure 1**).

Le brin sens est rendu fluorescent par l'amplification PCR utilisant une amorce modifiée en 5' par un groupe fluorescent (**Figure 1A****,** bande (A)). La bande (F) révélée en fluorescence (**Figure 1A**) correspond à la révélation d'amorces fluorescentes en excès. La discrimination entre les brins sens et antisens est faire grâce à l'utilisation d'une amorce d'amplification alourdie en 5' par polyadénylation et pégylation. Sur la **Figure 1B****,** gel de migration coloré au bleu de méthylène, on voit ainsi la bande correspondant au brin alourdi (A), la bande sens (F) et la bande d'amorces en excès (E).

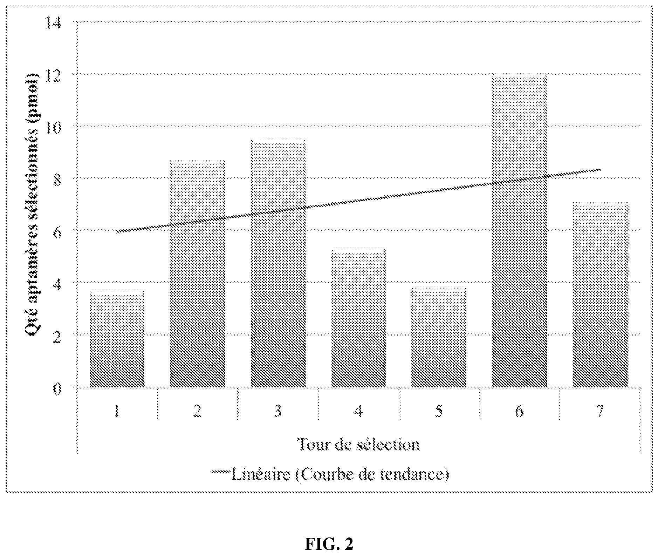

La quantité d'aptamères récupérée après découpage de la bande et purification à chaque tour de sélection est estimée par mesure de l'absorbance à 260 nm. L'évolution de cette quantité rend compte de l'enrichissement de la banque en aptamères spécifiques de la cible, enrichissement matérialisé par la courbe de tendance (**Figure 2**). La **Figure 2** montre une évolution apparemment très dispersée, mais la courbe de tendance indique qu'il y a un enrichissement d'un facteur 1,33. Les aptamères sont clonés puis séquencés.

Les séquences suivantes d'aptamères ont été sélectionnées :

Apta1 :5’ATACCAGCTTATTCAATTTATCCGCAACCCACCTAGCGCCCTACC
TCGTGGGAATCCAAACCCAACCAGTCCACCCACAGATAGTAAGTGCAAT
CT-3’ (SEQ ID NO : 3)

Apta2 :5'ATACCAGCTTATTCAATTGTGTCTGTTCACTCGTCGATACACAGC
ATACTCAACCCAGGCCCCTGACTGCTAATCCCCAGATAGTAAGTGCAAT
CT-3' (SEQ ID NO : 4)

Apta3 :5’ATACCAGCTTATTCAATTGTGTCTGTTCACTCGTCGATACACAAC
ATACTCAACCCAGGCCCCTGACTGCTAATCCCCAGATAGTAAGTGCAAT
CT-3’ (SEQ ID NO : 58)

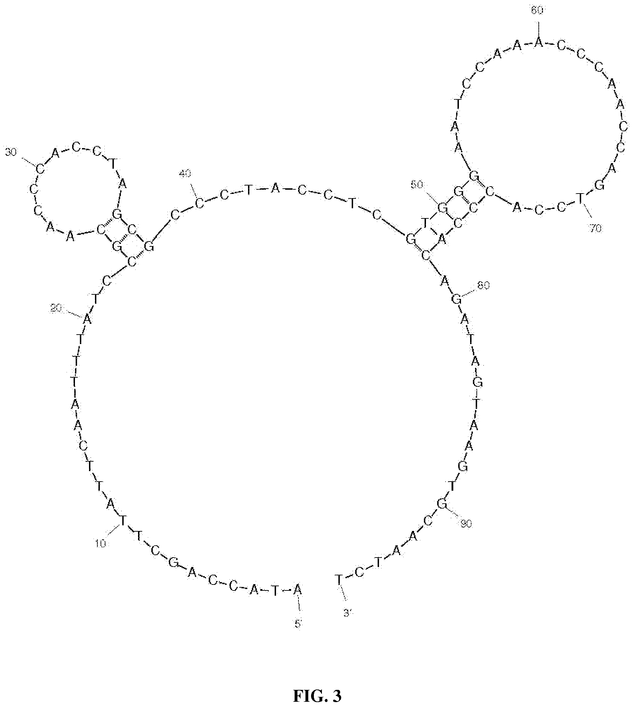

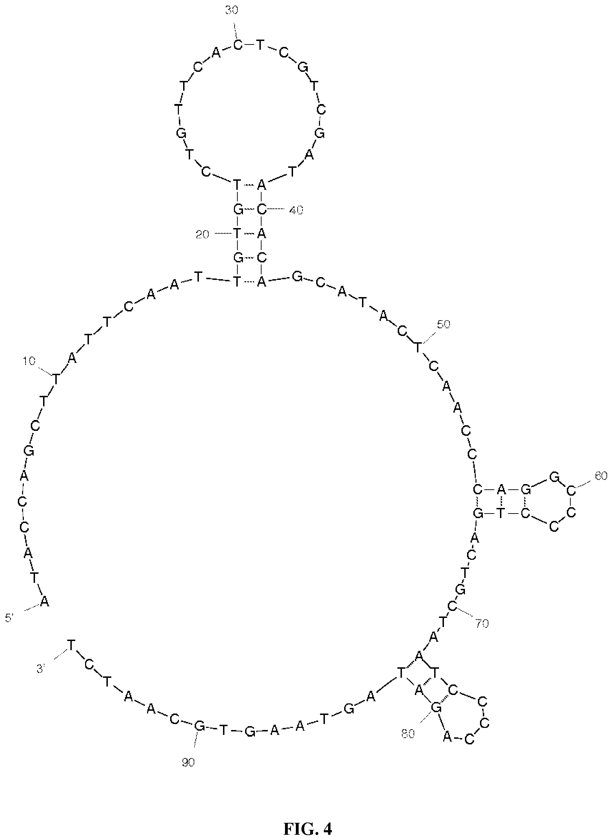

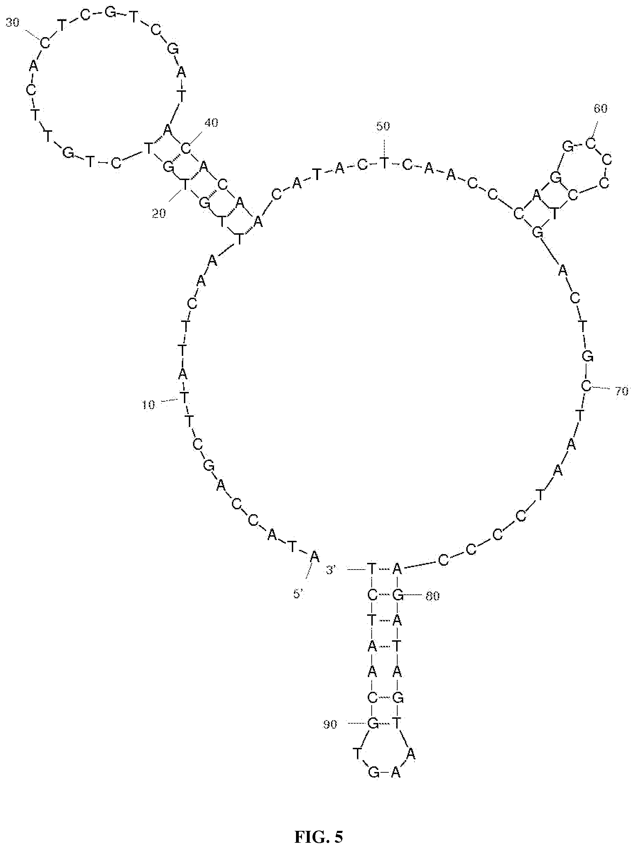

La modélisation de ces molécules par le logiciel mfold donne la structure représentée (**Figure 3** pour Apta 1, **Figure 4** pour Apta 2 et **Figure 5** pour Apta 3).

La structure secondaire des aptamères sélectionnés est réalisée à l'aide du logiciel mfold disponible par exemple sur le site du laboratoire Michael Zuker : http://bioinfo.math.rpi.edu/∼zukerm ou à l'adresse suivante : http://mfold.rna.albany.edu/?q=mfold/download-mfold. L'algorithme utilisé par ce logiciel est également basé sur les recherches décrites dans Mathews et al. (1999. J Mol Biol. 288(5):911-940). La structure de Apta 1 est présentée **Figure 3****.** La structure de Apta 2 est présentée **Figure 4****.** La structure de Apta 3 est présentée **Figure 5****.**

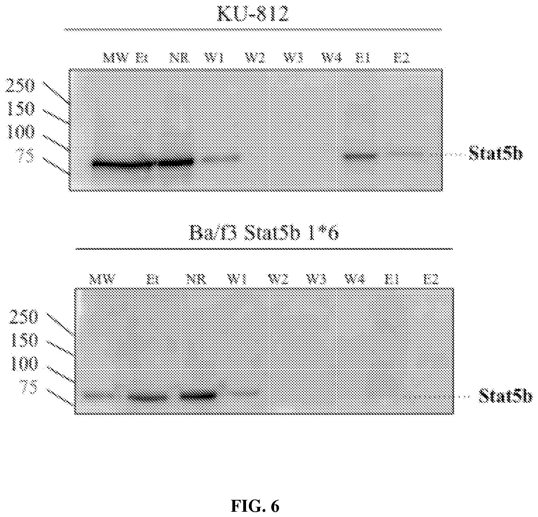

L'activité de l'aptamère Apta1 est testée par Western Blot sur les STAT5 cellulaires (issues du cytoplasme et du noyau). La validation de la complémentarité des oligonucléotides sélectionnés pour les facteurs de transcription STAT5B natifs est effectué par une expérience de pull down dans les cellules KU-812 et les cellules Ba/f3 STAT5B 1^{∗}6 (**Figure 6**). Cette expérience montre bien qu'Aptal reconnait la protéine STAT5 cellulaire, alors qu'il a été sélectionné contre la protéine STAT5B recombinante.

### Exemple 3 : Effets de Apta1 sur les lignées cellulaires

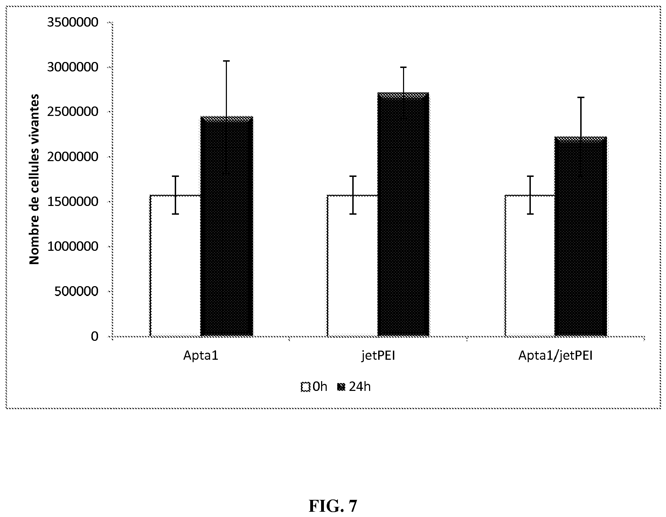

La croissance des cellules KU-812 transfectées par Apta1 est présentée sur la **figure 7****.**

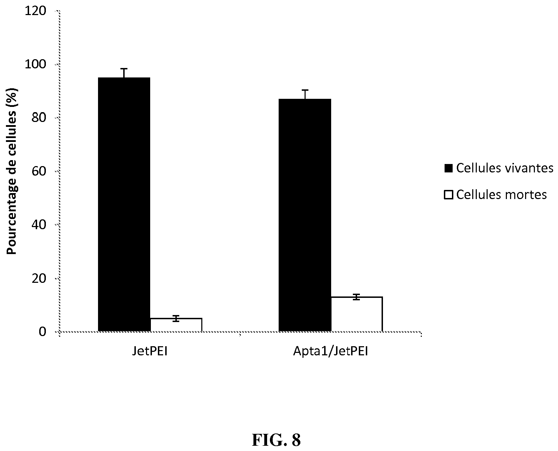

La mesure de la croissance montre une baisse du nombre de cellules en présence d'Apta1 transfecté à l'aide de JetPEI. Considérant que les brins d'ADN sont très rapidement dégradés (quelques heures) par les nucléases cellulaires, en particulier nucléaires, cette baisse, même légère, indique la possibilité qu'Aptal régule négativement l'activité leucémogène de STAT5. Cette hypothèse est d'autant plus valable que l'effet mesuré est systématiquement observé au cours d'expériences menées indépendamment (différentes décongélations, différents expérimentateurs, comptages à l'aveugle). De plus Apta1 a un effet sur la mortalité cellulaire **(****Figure 8****).**

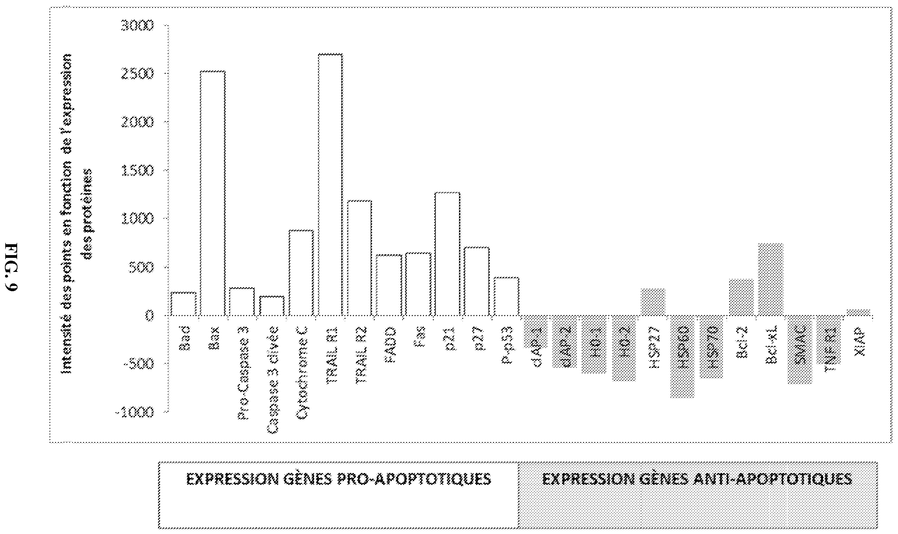

L'effet sur les gènes impliqués dans l'apoptose est ensuite mesuré **(****Figure 9****).** D'après ces mesures, Apta1 augmente de façon significative l'activité des gènes qui promeuvent l'apoptose et diminue également l'expression de gènes anti-apoptotiques des cellules leucémiques.

En résumé, Apta1 reconnait la protéine STAT5B recombinante, la protéine STAT5 extraite des cellules, diminue la croissance cellulaire des cellules leucémiques et régule l'expression des gènes impliqués dans l'apoptose.

### Exemple 4 : Effets de Apta2 sur la croissance des lignées cellulaires :

Les cellules KU-812 ont été transfectées avec Apta2. La croissance des cellules KU-812 transfectées par Apta2 est présentée sur la **Figure 10****.** Apta2 réduit la croissance des cellules KU-812.

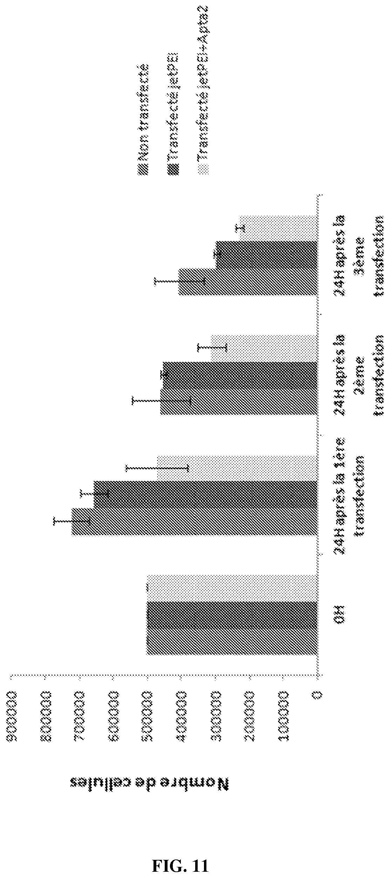

Considérant que l'effet d'Apta2 ne peut qu'être transitoire au vu du temps de ½ vie d'un brin d'ADN simple brin dans une cellule (soit 1 à 3 heures), nous avons exploré l'effet cumulé d'Apta2 lors de transfections successives, effectuées toutes les 24 heures **(****Figure 11****).** Après 24 heures, les cellules sont comptées puis transfectées de nouveau, dans les mêmes conditions. Ce procédé est répété trois fois.

On observe que les cellules transfectées avec Apta2 présentent systématiquement une croissance inférieure à celle des cellules non transfectées ou traitées avec JetPEI seul, ce qui confirme l'effet précédemment observé. En revanche, les cellules sont en souffrance au bout de 48 heures (2^{ème} transfection), elles ne poussent plus même dans le puits contrôle (sans transfection).

### Exemple 5 : Effets de Apta2 sur la viabilité des lignées cellulaires :

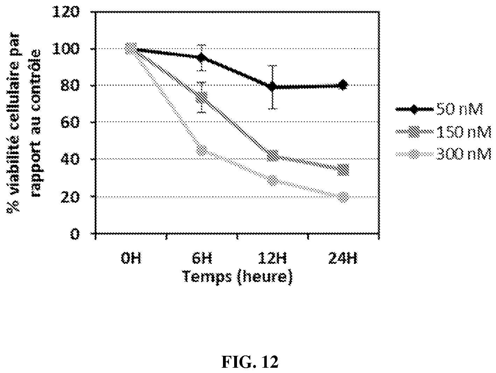

Les cellules KU812 ont été transfectées par différentes concentrations d'Apta2: 50 nM, 150 nM et 300 nM pour tester la dose-dépendance **(****Figure 12****).** La viabilité cellulaire a été ensuite étudiée en fonction du temps. Pour cela, un comptage cellulaire a été réalisé, pour chacune de ces concentrations après 6 heures, 12 heures et 24 heures de transfection.

Les résultats montrent un effet significatif d'apta2 en fonction du temps et de la dose d'aptamère. On observe une diminution statiquement significative du nombre de cellules vivantes après dès 6 heures de transfection par 150 nM d'Apta2. Le calcul du pourcentage des cellules vivantes par rapport à la condition contrôle montrent que seulement 20% de cellules restent vivantes après 24 heures de transfection par 9 µg (300 nM) d'Apta2.

### Exemple 6 : Effets de Apta2 sur l'apoptose des lignées cellulaires :

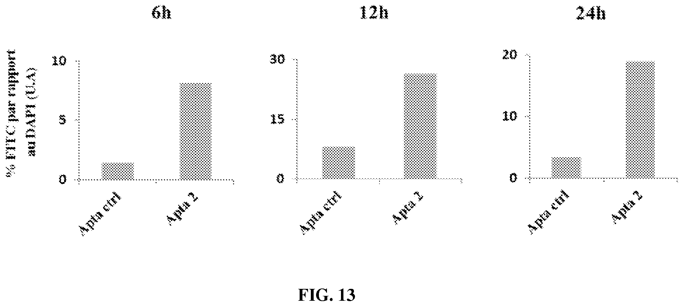

L'observation de la fragmentation de l'ADN après transfection a été réalisée par marquage des noyaux au DAPI puis par marquage des fragments au niveau des extrémités 3'OH libre aux dUTP couplés à la fluorescéine. L'analyse par TUNEL a été effectuée sur les cellules qui ont été traitées par 150 nM d'apta2 et d'apta ctrl (séquence Apta2 dégénérée) après 6 heures, 12 heures et 24 heures de transfection ((**Figure 13**).

Les résultats du TUNEL en présence d'Apta2 montrent une augmentation de l'intensité de la fluorescence due au FITC dans le cas où les cellules sont transfectées par Apta2. Cette fluorescence devient plus forte au cours de temps indiquant une augmentation du nombre de cellules mortes et confirmant ainsi les résultats de l'étude de la viabilité cellulaire. L'intensité de la FITC a été quantifiée grâce au logiciel Image Studio Lite.

## Revendications

1. Aptamère d'acides nucléiques se liant spécifiquement à STAT5, de préférence à STAT5B, ledit aptamère étant **caractérisé en ce qu'**il comprend la séquence SEQ ID NO : 1, SEQ ID NO : 2 ou SEQ ID NO : 57, ou un variant ayant au moins 90% d'identité de séquence avec SEQ ID NO : 1, SEQ ID NO: 2 ou SEQ ID NO : 57.

2. Aptamère d'acides nucléiques se liant spécifiquement à STAT5 selon la revendication 1, comprenant en outre un groupement additionnel de stabilisation et/ou un groupement additionnel pour vectorisation.

3. Composition pharmaceutique comprenant au moins un aptamère selon la revendication **1** ou **2** et un excipient pharmaceutiquement acceptable.

4. Médicament comprenant au moins un aptamère selon la revendication **1** ou **2.**

5. Aptamère d'acides nucléiques selon la revendication **1** ou **2,** composition pharmaceutique selon la revendication **3,** ou médicament selon la revendication **4,** pour son utilisation dans le traitement de cancers, de préférence de leucémies.

6. Aptamère d'acides nucléiques, composition pharmaceutique ou médicament pour son utilisation selon la revendication **5,** en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, les inhibiteurs des tyrosines kinases, les agents différenciants, ou un mélange de ceux-ci.

7. Aptamère d'acides nucléiques selon la revendication **1** ou **2,** en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, les inhibiteurs des tyrosines kinases, les agents différenciants, ou un mélange de ceux-ci.

8. Composition pharmaceutique selon la revendication **3,** ou médicament selon la revendication **4,** en combinaison avec un autre agent actif sélectionné parmi les agents anti-cancéreux, les agents anti-angiogéniques, les agents anti-métastatiques, les agents anti-leucémiques, les agents anti-foliques, les agents anti-métabolites, les agents alkylants, les agents intercalants, les agents agissant sur le fuseau mitotique, les inhibiteurs des tyrosines kinases, les agents différenciants, ou un mélange de ceux-ci.

9. Procédé de détection de STAT5, de préférence de STAT5B, dans un échantillon biologique comprenant :
a. la mise en contact de l'aptamère selon la revendication **1** ou **2** avec ledit échantillon préalablement prélevé sur un sujet,
b. la détermination de la quantité dudit aptamère lié audit échantillon.

## Patentansprüche

1. Nukleinsäure-Aptamer, das sich spezifisch an STAT5, vorzugsweise an STAT5B, bindet, wobei das Aptamer **dadurch gekennzeichnet ist, dass** es die Sequenz SEQ ID NO: 1, SEQ ID NO: 2 oder SEQ ID NO: 57 oder eine Variante mit mindestens 90% Sequenzidentität mit SEQ ID NO: 1, SEQ ID NO: 2 oder SEQ ID NO: 57 umfasst.

2. Nukleinsäure-Aptamer, das sich spezifisch an STAT5 bindet, nach Anspruch **1,** weiterhin umfassend eine zusätzliche Stabilisierungsgruppe und/oder eine zusätzliche Gruppe zur Vektorisierung.

3. Pharmazeutische Zusammensetzung, umfassend mindestens ein Aptamer nach Anspruch **1** oder **2** und einen pharmazeutisch annehmbaren Hilfsstoff.

4. Medikament, umfassend mindestens ein Aptamer nach Anspruch **1** oder **2.**

5. Nukleinsäure-Aptamer nach Anspruch **1** oder **2,** pharmazeutische Zusammensetzung nach Anspruch **3** oder Medikament nach Anspruch **4** zu dessen bzw. deren Verwendung bei der Behandlung von Krebserkrankungen, vorzugsweise Leukämien.

6. Nukleinsäure-Aptamer, pharmazeutische Zusammensetzung oder Medikament zu dessen bzw. deren Verwendung nach Anspruch **5** in Kombination mit einem anderen aktiven Mittel, ausgewählt aus Antikrebsmitteln, antiangiogenen Mitteln, antimetastatischen Mitteln, anti-Leukämie-Mittel, Antifolienmittel, Antimetabolitenmittel, Alkylierungsmitteln, Interkalierungsmitteln, auf den Spindelapparat einwirkenden Mitteln, Hemmern von Tyrosinkinasen, Differenzierungsmitteln, oder einem Mischung davon.

7. Nukleinsäure-Aptamer nach Anspruch **1** oder **2** in Kombination mit einem anderen aktiven Mittel, ausgewählt aus Antikrebsmitteln, antiangiogenen Mitteln, antimetastatischen Mitteln, anti-Leukämie-Mittel, Antifolienmittel, Antimetabolitenmittel, Alkylierungsmitteln, Interkalierungsmitteln, auf den Spindelapparat einwirkenden Mitteln, Hemmern von Tyrosinkinasen, Differenzierungsmitteln, oder einem Mischung davon.

8. Pharmazeutische Zusammensetzung nach Anspruch **3** oder Medikament nach Anspruch **4** in Kombination mit einem anderen aktiven Mittel, ausgewählt aus Antikrebsmitteln, antiangiogenen Mitteln, antimetastatischen Mitteln, anti-Leukämie-Mittel, Antifolienmittel, Antimetabolitenmittel, Alkylierungsmitteln, Interkalierungsmitteln, auf den Spindelapparat einwirkenden Mitteln, Hemmern von Tyrosinkinasen, Differenzierungsmitteln, oder einem Mischung davon.

9. Verfahren zum Nachweis von STAT5, vorzugsweise STAT5B, in einer biologischen Probe, umfassend:
a. inkontaktbringen des Aptamers nach Anspruch **1** oder **2** mit der Probe, die zuvor von einem Probanden genommen wurde,
b. bestimmen der Menge des mit der Probe gebundenen Aptamers.

## Claims

1. Nucleic acid aptamer binding specifically to STAT5, preferably to STAT5B, said aptamer being caracterised in that it comprises the sequence SEQ ID NO: 1, SEQ ID NO: 2 or SEQ ID NO: 57, or a variant having at least 90% of sequence identity with SEQ ID NO: 1, SEQ ID NO: 2 or SEQ ID NO: 57.

2. Nucleic acids aptamer binding specifically to STAT5 according to claim **1,** further comprising an additional stabilisation group and/or additional vectorisation group.

3. A pharmaceutical composition comprising at least one aptamer according to claim **1** or **2** and a pharmaceutically acceptable excipient.

4. A medicament comprising at least one aptamer according to claim **1** or **2.**

5. Nucleic acid aptamer according to claim **1** or **2,** pharmaceutical composition according to claim **3,** or medicament according to claim **4,** for its use in the treatment of cancers, preferably of leukemias.

6. Nucleic acids aptamer, pharmaceutical composition or medicament for its use according to claim **5,** in combination with another active agent selected among anti-cancer agents, anti-angiogenic agents, anti-metastatic agents, anti-leukemic agents, anti-folic agents, anti-metabolic agents, alkylating agents, intercalating agent, agents acting on mitotic spindle, tyrosine-kinase inhibitors, differentiating agents, or a mixture thereof.

7. Nucleic acid aptamer according to claim **1** or **2,** in combination with another active agent selected among anti-cancer agents, anti-angiogenic agents, anti-metastatic agents, anti-leukemic agents, anti-folic agents, anti-metabolic agents, alkylating agents, intercalating agent, agents acting on mitotic spindle, tyrosine-kinase inhibitors, differentiating agents, or a mixture thereof.

8. Pharmaceutical composition according to claim **3,** or medicament according to claim **4,** in combination with another active agent selected among anti-cancer agents, anti-angiogenic agents, anti-metastatic agents, anti-leukemic agents, anti-folic agents, anti-metabolic agents, alkylating agents, intercalating agent, agents acting on mitotic spindle, tyrosine-kinase inhibitors, differentiating agents, or a mixture thereof.

9. Process for the detection of STAT5, preferably of STAT5B, in a biological sample comprising:
a. contacting the aptamer according to claim **1** or **2** with said sample previously collected on a subject,
b. determining the quantity of said aptamer bound to said sample.
